# EUROPÄISCHE PATENTANMELDUNG

(11) **EP 2 382 961 A2**
(43) Veröffentlichungstag der Anmeldung: **02.11.2011**
(21) Anmeldenummer: 11160998.8
(22) Anmeldetag: 04.04.2011
(51) Int. Cl.: A61K 8/06, A61K 8/895, A61Q 19/08

(54) **Kosmetische Zusammensetzung zur optischen Kaschierung von Falten**

(30) Priorität: 28.04.2010 DE 102010028313
(71) Anmelder: Henkel AG & Co. KGaA, 40589 Düsseldorf (DE)
(72) Erfinder: Stadler, Iris Marina, 47249, Duisburg (DE); Bialasinski, Leszek, 40883, Ratingen (DE); Waldmann-Laue, Marianne, 40789, Monheim (DE)

(57) **Zusammenfassung**

Gegenstand der vorliegenden Anmeldung sind ausgewählte Öl-in-Wasser-Emulsionen mit einem Gehalt an Siliconelastomeren, die sich hervorragend zur optischen Kaschierung von Falten eignen.

## Beschreibung

Gegenstand der vorliegenden Anmeldung sind ausgewählte Öl-in-Wasser-Emulsionen mit einem Gehalt an Siliconelastomeren, die sich hervorragend zur optischen Kaschierung von Falten eignen.

### Beschreibung

Moderne kosmetische Zusammensetzungen zur Pflege der Haut, insbesondere der Gesichtshaut, müssen vielfachen Anforderungen des Verbrauchers genügen.

Neben der Erfüllung ihrer eigentlichen Bestimmung - Feuchtigkeitsbindung, Stärkung der Barrierefunktion der Haut und Verbesserung des Erscheinungsbildes der Haut bei Hautcremes, -gelen, -lotionen, -masken usw. - werden weitere kosmetische Effekte erwartet. Bei einer Hautcreme oder einer ähnlichen Zusammensetzung kann das die Faltenbehandlung, Hautaufhellung oder Hautbräunung, das Kaschieren von Hautunreinheiten und Hautunebenheiten sein.

Ein weiteres wichtiges Kriterium bei der Beurteilung einer kosmetischen Zusammensetzung durch den Anwender ist der sensorische, insbesondere der taktil-sensorische Eindruck, der bei der Applikation der Zusammensetzung auf die Haut hervorgerufen wird. Als besonders angenehm wird von vielen Verbrauchern der sensorische Eindruck, den Silicon-Verbindungen, z. B. Siliconöle oder Siliconelastomere erzeugen, empfunden. Siliconöle werden aufgrund ihres Spreit-und Einzugsverhaltens als samtweich und nicht-fettend empfunden. Sie hinterlassen die Haut ohne Fettglanz und wirken nicht comedogen.

Ein zu hoher Anteil an Siliconölen in einer kosmetischen Zusammensetzung wird allerdings von vielen Verbrauchern nach einer gewissen Zeit als zu wenig Haut pflegend, mitunter sogar als austrocknend, empfunden. Es hat sich hier als günstig erweisen, Siliconöl-Wasser-Emulsionen zu formulieren. Die Emulgierung der Siliconöle zusammen mit Wasser hat weitere Vorteile: zahlreiche Wirkstoffe gegen die Hautalterung oder andere negative Hautzustände sind eher wasserlöslich als öllöslich und daher besser oder überhaupt nur mit einer wasserhaltigen Zusammensetzung kompatibel. Nicht zuletzt stellt Wasser einen ökonomisch günstigen Rohstoff dar. Durch den Zusatz von Wasser wird allerdings der taktil-sensorische Eindruck, der direkt bei der Applikation der Zusammensetzung auf die Haut hervorgerufen wird, negativ beeinträchtigt. Außerdem sieht sich der Fachmann bei der Herstellung von Emulsionen, thermodynamisch instabilen Systemen, immer mit einem Stabilitätsproblem konfrontiert, für das er eine optimale Lösung finden muss.

Weiterhin haben in den letzten Jahren verstärkt Hautkosmetika an Bedeutung gewonnen, die die Alterungserscheinungen der Haut, wie Fältchen, Falten und Altersflecken, optisch kaschieren, beispielsweise durch so genannte Soft Focus-Pigmente. Hierzu geeignete Rohstoffe sind insbesondere Siliconelastomere.

EP 1097703 A1 offenbart eine kosmetische Zusammensetzung zur Gesichtspflege, die als Öl-in-Wasser-Emulsion formuliert ist und eine wässrige Siliconelastomer-Dispersion enthält.

EP 1550432 A1 offenbart einen wasserhaltigen Gesichtspuder, der eine wässrige Siliconelastomer-Dispersion sowie weitere partikuläre Bestandteile enthält.

EP 1097695 A1 offenbart eine kosmetische Zusammensetzung zur Gesichtspflege, die als Wasser-in-Öl-Emulsion formuliert ist und eine wässrige Siliconelastomer-Dispersion sowie ein Silicon-basiertes Siliconelastomergel enthält.

EP 1064930 offenbart eine kosmetische Zusammensetzung zur Gesichtspflege, die eine wässrige Siliconelastomer-Dispersion sowie ein Silicon-basiertes Siliconelastomergel enthält.

Auch US 56689345 und WO 2006/115816 A1 offenbaren kosmetische Zusammensetzungen zur Kaschierung von Falten, die eine wässrige Siliconelastomer-Dispersion enthalten.

Die kaschierende oder abdeckende Wirkung dieser Zusammensetzungen hängt aber nicht nur vom Siliconelastomergehalt ab, sondern lässt sich auch durch die Basisformulierung beeinflussen.

### Aufgabenstellung

Eine Aufgabe der vorliegenden Erfindung war die Bereitstellung einer kosmetischen Zusammensetzung mit hervorragenden Eigenschaften zur optischen Kaschierung von Falten. Eine weitere Aufgabe der vorliegenden Erfindung war die Bereitstellung einer kosmetischen Zusammensetzung mit hervorragenden pflegenden und feuchthaltenden Eigenschaften. Eine weitere Aufgabe der vorliegenden Erfindung war die Bereitstellung einer sensorisch, insbesondere taktil-sensorisch, ansprechenden kosmetischen Zusammensetzung. Eine weitere Aufgabe der vorliegenden Erfindung war die Bereitstellung einer lager- und/oder temperaturstabilen kosmetischen Zusammensetzung auf Basis einer wasser- und ölhaltigen Zusammensetzung, die größere Mengen einer dispergierten Partikelphase enthält.

Überraschend wurde nun gefunden, dass durch die anspruchsgemäße Kombination an Inhaltsstoffen besonders lager- und temperaturstabile kosmetische Zusammensetzungen in Form von Öl-in-Wasser-Emulsionen mit einem außergewöhnlich angenehmen sensorischen, insbesondere taktil-sensorischen, Eindruck hergestellt werden können, die eine sofort einsetzende hervorragende Faltenkaschierung bewirken.

Ein erster Gegenstand der vorliegenden Erfindung ist eine kosmetische Zusammensetzung in Form einer Öl-in-Wasser-Emulsion, die, jeweils bezogen auf das Gesamtgewicht der Zusammensetzung, folgende Bestandteile enthält:
a) 6 - 12 Gew.-% Ölphase, darin
   a)i) 0,1 - 0,7 Gew.-% Siliconelastomer, das die Ölphase verdickt,
   a)ii) 0 bis maximal 4 Gew.-% Siliconöl,
b) 13 - 35 Gew.-% dispergierte Partikelphase, darin
   b)i) 13 - 35 Gew.-% Siliconelastomer, das in der wässrigen Phase oder in der Emulsion dispergiert vorliegt und nicht die Ölphase verdickt,
c) 52,4 - 80,95 Gew.-% wässrige Phase,
d) wobei die Zusammensetzung insgesamt 0,05 bis 0,6 Gew.-% Emulgator(en) enthält, und
e) wobei die Zusammensetzung frei ist von Wachsen, Alkanolen und Polyolestern, die jeweils einen Schmelzpunkt von 30°C und darüber aufweisen.

Die erfindungsgemäßen kosmetischen Zusammensetzungen liegen in Form einer Öl-in-Wasser-Emulsion vor, die, bezogen auf das Gesamtgewicht der Zusammensetzung, 6 - 12 Gew.-% Ölphase enthält. Überraschend wurde festgestellt, dass ein höherer Gehalt an Ölphase die Falten kaschierende Wirkung beeinträchtigt. Ein niedrigerer Gehalt an Ölphase beeinträchtigt die haptischen und sensorischen Eigenschaften der Zusammensetzung und ihr Hautgefühl nach der Applikation. Bevorzugt ist ein Gehalt an Ölphase von 7 - 11 Gew.-%, besonders bevorzugt 8 - 10 Gew.-%, jeweils bezogen auf das Gesamtgewicht der Zusammensetzung. Zur Ölphase zählen die kosmetischen Öle sowie öllösliche Wirk- und Hilfsstoffe, wie Antioxidantien, z. B. Tocopherole, Parfüm oder öllösliche UV-Filter, z. B. Octocrylen oder Butylmethoxydibenzoylmethan.

Eventuell enthaltene lipophile Emulgatoren werden erfindungsgemäß nicht zur Ölphase gerechnet.

Die Ölphase kann ein Siliconöl oder auch mehrere Siliconöle enthalten, allerdings ist der Silicon-ölgehalt beschränkt auf maximal 4 Gew.-%, bezogen auf das Gesamtgewicht der Zusammensetzung. Überraschend wurde festgestellt, dass ein höherer Gehalt an Siliconölen zu einem trockenen, stumpfen Hautgefühl bei und nach der Applikation führt, das erfindungsgemäß unerwünscht war. Bevorzugt ist ein Gehalt an Siliconöl(en) von 0,5 - 3 Gew.-%, besonders bevorzugt 1 - 2,5 Gew.-%, jeweils bezogen auf das Gesamtgewicht der Zusammensetzung.

Erfindungsgemäß bevorzugte Siliconöle sind ausgewählt aus Dialkyl- und Alkylarylsiloxanen, wie beispielsweise Cyclotetrasiloxan, Cyclopentasiloxan, Cyclohexasiloxan, Dimethylpolysiloxan und Methylphenylpolysiloxan, aber auch Hexamethyldisiloxan, Octamethyltrisiloxan und Decamethyltetrasiloxan zählen. Besonders bevorzugt sind flüchtige Siliconöle, die cyclisch sein können, wie z. B. Octamethylcyclotetrasiloxan, Decamethylcyclopentasiloxan und Dodecamethylcyclohexasiloxan sowie Mischungen hiervon, wie sie z. B. in den Handelsprodukten DC 244, 245, 344 und 345 von Dow Corning enthalten sind. Ebenfalls besonders bevorzugt sind flüchtige lineare Siliconöle, insbesondere Hexamethyldisiloxan (L_{z}), Octamethyltrisiloxan (L₃), Decamethyltetrasiloxan (L₄) sowie beliebige Zweier- und Dreiermischungen aus L₂, L₃ und/oder L₄, bevorzugt solche Mischungen, wie sie z. B. in den Handelsprodukten DC 2-1184, Dow Corning^{®} 200 (0,65 cSt) und Dow Corning^{®} 200 (1,5 cSt) von Dow Corning enthalten sind. Ebenfalls bevorzugt ist Phenyl Trimethicone, insbesondere niedermolekulares Phenyl Trimethicone mit einem Dampfdruck bei 20°C von etwa 2000 Pa, wie es beispielsweise von GE Bayer Silicones/Momentive unter dem Namen Baysilone Fluid PD 5 erhältlich ist.

Unter flüchtigen Ölen versteht man erfindungsgemäß solche Öle, die bei 20 °C und einem Umgebungsdruck von 1013 hPa einen Dampfdruck von 2,66 Pa - 40000 Pa (0,02 mm - 300 mm Hg), bevorzugt 13 - 12000 Pa (0,1 - 90 mm Hg), besonders bevorzugt 15 - 8000 Pa, außerordentlich bevorzugt 300 - 3000 Pa, aufweisen. Unter nicht-flüchtigen Ölen versteht man erfindungsgemäß solche Öle, die bei 20 °C und einem Umgebungsdruck von 1013 hPa einen Dampfdruck von weniger als 2,66 Pa (0,02 mm Hg) aufweisen.

Erfindungsgemäß bevorzugte Zusammensetzungen sind dadurch gekennzeichnet, dass sie mindestens ein Esteröl enthalten. Bevorzugt ist ein Gesamtgehalt an Esteröl(en) von 5,9 - 11,9 Gew.-%, besonders bevorzugt 6,5 - 10 Gew.-%, außerordentlich bevorzugt 8 - 9 Gew.-%, jeweils bezogen auf das Gesamtgewicht der Zusammensetzung.

Besonders bevorzugte Esteröle sind die Benzoesäureester von linearen oder verzweigten C₈₋₂₂-Alkanolen. Außerordentlich bevorzugt sind Benzoesäure-C₁₂-C₁₅-Alkylester, z. B. erhältlich als Handelsprodukt Finsolv^{®} TN (C₁₂-C₁₅-Alkylbenzoal), mit dem besonders hervorragende Faltenkaschiereffekte erzielt werden können. Weitere bevorzugte Benzoate sind Benzoesäureisostearylester, z. B. erhältlich als Finsolv^{®} SB, Ethylhexylbenzoat, z. B. erhältlich als Finsolv^{®} EB, und Benzoesäureoctyldocecylester, z. B. erhältlich als Finsolv^{®} BOD. Weiterhin erfindungsgemäß bevorzugt sind Ester der linearen oder verzweigten gesättigten oder ungesättigten Fettalkohole mit 2 - 30 Kohlenstoffatomen mit linearen oder verzweigten gesättigten oder ungesättigten Fettsäuren mit 2 - 30 Kohlenstoffatomen, die hydroxyliert sein können. Besonders bevorzugte Esteröle sind ausgewählt aus den Estern der linearen oder verzweigten gesättigten Fettalkohole mit 2 - 5 Kohlenstoffatomen mit linearen oder verzweigten gesättigten oder ungesättigten Fettsäuren mit 10 - 18 Kohlenstoffatomen, die hydroxyliert sein können. Bevorzugte Beispiele hierfür sind Isopropylpalmitat, Isopropylstearat, Isopropylmyristat, Hexyldecylstearat, Hexyldecyllaurat, Isononylisononanoat, 2-Ethylhexylpalmitat und 2-Ethylhexylstearat. Ebenfalls bevorzugt sind Isooctylstearat, Isononylstearat, Isocetylstearat, Isononylisononanoat, Isotridecylisononanoat, Cetearylisononanoat, 2-Ethylhexyllaurat, 2-Ethylhexylisostearat, 2-Ethylhexylcocoat, 2-Octyldodecylpalmitat, Butyloctansäure-2-butyloctanoat, Diisotridecylacetat, n-Hexyllaurat, n-Decyloleat, Oleyloleat, Oleylerucat, Erucyloleat, C₁₂-C₁₅-Alkyllactat und Di-C₁₂-C₁₃-Alkylmalat.

Weitere kosmetische Öle, die erfindungsgemäß bevorzugt sein können, sind ausgewählt aus flüssigen Paraffinölen, Isoparaffinölen und synthetischen Kohlenwasserstoffen, wie 1,3-Di-(2-ethyl-hexyl)-cyclohexan (Cetiol^{®} S), weiterhin ausgewählt aus Di-n-alkylethern mit insgesamt 12 bis 36, insbesondere 12 bis 24 C-Atomen, wie insbesondere Di-n-octylether, Di-n-decylether, Din-nonylether, Di-n-undecylether, n-Hexyl-n-octylether und n-Octyl-n-decylether. 1,3-Di-(2-ethylhexyl)-cyclohexan (Cetiol^{®} S) und Di-n-octylether (Cetiol^{®} OE) können besonders bevorzugt sein. Weiterhin können symmetrische, unsymmetrische oder cyclische Ester der Kohlensäure mit Fettalkoholen, insbesondere Dicaprylylcarbonat (Cetiol^{®} CC), besonders bevorzugt sein.

Weitere erfindungsgemäß bevorzugte Öle sind ausgewählt aus verzweigten gesättigten oder ungesättigten Fettalkoholen mit 6 - 30 Kohlenstoffatomen. Diese Alkohole werden häufig auch als Guerbet-Alkohole bezeichnet, da sie nach der Guerbet-Reaktion erhältlich sind. Bevorzugte Alkoholöle sind 2-Hexyldecanol, 2-Octyldodecanol und 2-Ethylhexylalkohol.

Die Ölphase der erfindungsgemäßen Zusammensetzungen enthält, bezogen auf das Gesamtgewicht der erfindungsgemäßen Zusammensetzung, eine Gesamtmenge von 0,1 - 0,7 Gew.-% mindestens eines Siliconelastomers, das die Ölphase verdickt. Bevorzugt sind 0,2 - 0,6 Gew.-%, außerordentlich bevorzugt 0,3 - 0,4 Gew.-%, jeweils bezogen auf das Gesamtgewicht der erfindungsgemäßen Zusammensetzung, mindestens eines Siliconelastomers, das die Ölphase verdickt, enthalten. Ein derartiges Siliconelastomer führt zu hervorragenden sensorischen Eigenschaften und unterstützt weiterhin das Faltenkaschiervermögen der erfindungsgemäßen Zusammensetzung.

Eine bevorzugte Ausführungsform der Erfindung ist dadurch gekennzeichnet, dass das mindestens eine Ölphasen-verdickende Siliconelastomer erhältlich ist durch die Vernetzung eines Organopolysiloxans, das mindestens 2 C₂ - C₁₀-Alkenylgruppen mit terminaler Doppelbindung in jedem Molekül enthält, mit einem Organopolysiloxan, das mindestens zwei Silicon-gebundene Wasserstoffatome in jedem Molekül aufweist.

Erfindungsgemäß besonders bevorzugte Organopolysiloxane mit mindestens 2 C₂ - C₁₀ - Alkenyl-Gruppen mit terminaler Doppelbindung im Molekül sind ausgewählt aus Methylvinylsiloxanen, Methylvinylsiloxan-Dimethylsiloxan-Copolymeren, Dimethylpolysiloxanen mit Dimethylvinylsiloxy-Endgruppen, Dimethylsiloxan-Methylphenylsiloxan-Copolymeren mit Dimethylvinylsiloxy-Endgruppen, Dimethylsiloxan-Diphenylsiloxan-Methylvinylsiloxan-Copolymeren mit Dimethylvinylsiloxy-Endgruppen, Dimethylsiloxan-Methylvinylsiloxan-Copolymeren mit Trimethylsiloxy-Endgruppen, Dimethylsiloxan-Methylphenylsiloxan-Methylvinylsiloxan-Copolymeren mit Trimethylsiloxy-Endgruppen, Methyl-(3,3,3-trifluoropropyl)-polysiloxanen mit Dimethylvinylsiloxy-Endgruppen und Dimethylsiloxan-Methyl-(3,3,3-trifluoropropyl)-siloxan-Copolymeren mit Dimethylvinylsiloxy-Endgruppen.

Erfindungsgemäß besonders bevorzugte vernetzende Organopolysiloxane mit mindestens zwei Silicon-gebundenen Wasserstoffatomen sind ausgewählt aus Methylhydrogenpolysiloxanen mit Trimethylsiloxy-Endgruppen, Dimethylsiloxan-Methylhydrogensiloxan-Copolymeren mit Trimethylsiloxy-Endgruppen und cyclischen Dimethylsiloxan-Methylhydrogensiloxan-Copolymeren.

Erfindungsgemäß besonders bevorzugte Siliconelastomere, die als Rohstoff bereits in einem bei Raumtemperatur unter Normalbedingungen flüssigen Öl vorgequollen vorliegen und ein Silicon-basiertes Gel darstellen, sind kommerziell erhältlich. Das Siliconelastomer kann sowohl in einem Siliconöl als auch in einem Nicht-Siliconöl vorgequollen sein.

Entsprechende Handelsprodukte sind beispielsweise SFE 168, ein Cyclomethicone (and) Dimethicone/Vinyl Dimethicone Crosspolymer von GE Silicones, Vinyl Dimethicone Crosspolymer, enthalten in KSG-15 (Cyclomethicone (and) Dimethicone/Vinyl Dimethicone Crosspolymer, Siliconelastomergehalt 4 - 10 Gew.-%), KSG-16 (Dimethicone (and) Dimethicone/Vinyl Dimethicone Crosspolymer, Siliconelastomergehalt 20 - 30 Gew.-%), KSG-17 (Cyclomethicone (and) Dimethicone/Vinyl Dimethicone Crosspolymer), KSG-18 (Phenyl Trimethicone (and) Dimethicone/ Phenyl Vinyl Dimethicone Crosspolymer, Siliconelastomergehalt 10 - 20 Gew.-%); and KSG-20, erhältlich von Shin Etsu, und von Grant Industries Inc. (Elmwood Park, NJ) die Produkte aus der Gransil^{®}-Serie, insbesondere Gransil SR-CYC (Cyclomethicone and Stearyl-Vinyl/hydromethylsiloxane Copolymer), Gransil^{®} RPS Gel (INCI-Bezeichnung: Cyclopentasiloxane and Polysilicone-11), Gransil^{®} GCM-4 (INCI-Bezeichnung: Cyclotetrasiloxane and Polysilicone-11), Gransil^{®}GCM-5 (INCI-Bezeichnung: Cyclopentasiloxane and Polysilicone-11), Gransil^{®} RPS (INCI-Bezeichnung: Cyclopentasiloxane and Polysilicone-11), GI-CD 10 (INCI-Bezeichnung: Cyclopentasiloxane (and) Stearoxymethicone/Dimethicone Copolymer (and) Dimethicone), Gransil^{®} IDS (INCI-Bezeichnung: Isododecane (and) Cyclotetrasiloxane (and) Polysilicone-11), Gransil^{®}PC-12 (INCI-Bezeichnung: Isododecane (and) Polysilicone-11), Gransil^{®}IDS-5 (INCI-Bezeichnung: Isododecane (and) Cyclopentasiloxane (and) Polysilicone-11), Gransil^{®}DMCM-5 (INCI-Bezeichnung: Dimethicone and Cyclopentasiloxane and Polysilicone-11), Gransil^{®}DMG-6 mit Dimethicone (6 cSt) (INCI-Bezeichnung: Dimethicone and Polysilicone-11), Gransil^{®}DMG-20 mit Dimethicone (20 cSt) (INCI-Bezeichnung: Dimethicone and Polysilicone-11), Gransil^{®} AM-8 Gel (INCI-Bezeichnung: Caprylyl Methicone and Cyclopentasiloxane and Polysilicone-11), Gransil^{®} DM 5 mit Dimethicone (5 cSt) (INCI-Bezeichnung: Dimethicone and Polysilicone-11), Gransil^{®} DMID (INCI-Bezeichnung: Dimethicone and Isododecane and Polysilicone-11), Gransil^{®} PM (INCI-Bezeichnung: Phenyl Trimethicone and Polysilicone-11), Gransil^{®} ININ (INCI-Bezeichnung: Isononyl Isononanoate (and) Polysilicone-11).

Ebenfalls mit Vorzug in den erfindungsgemäßen Zusammensetzungen einsetzbar sind Siliconelastomere, die als Rohstoff bereits in einem bei Raumtemperatur unter Normalbedingungen flüssigen Silicon, gemischt mit einem Nicht-Silicon-haltigen Öl vorgequollen vorliegen und ein Silicon-/Nicht-Silicon-basiertes Gel darstellen. Derartige Siliconelastomer-Zusammensetzungen sind ebenfalls kommerziell erhältlich, beispielsweise unter den Handelsnamen Gransil^{®} RJO (INCI-Bezeichnung: Cyclopentasiloxane and Polysilicone-11 and Jojoba Oil), Gransil^{®} OHS-5 (INCI-Bezeichnung: Cyclopentasiloxane and Polysilicone-11 and Octyl Hydroxystearate) und Gransil^{®} DML (INCI-Bezeichnung: Dimethicone (and) Neopentyl Glycol Diheptanoate (and) Polysilicone-11).

Eine außerordentlich bevorzugte Ausführungsform der Erfindung ist dadurch gekennzeichnet, dass das mindestens eine Ölphasen-verdickende Siliconelastomer durch die Vernetzung eines Organopolysiloxans, das mindestens 2 C₂ - C₁₀-Alkenylgruppen mit terminaler Doppelbindung in jedem Molekül enthält, mit mindestens einem alpha, omega-Dien erhältlich ist. Erfindungsgemäß besonders bevorzugte vernetzende alpha, omega-Diene weisen die Formel CH₂=CH(CH₂)ₓCH=CH₂ mit X = 1 - 20 auf. Besonders bevorzugte alpha, omega-Diene sind ausgewählt aus 1,4-Pentadien, 1,5-Hexadien, 1,6-Heptadien, 1,7-Octadien, 1,8-Nonadien, 1,11-Dodecadien, 1,13-Tetradecadien und 1,19- Eicosadien.

Entsprechende Handelsprodukte sind Dow Corning EL 8040 ID Silicone Organic Blend (eine Quellung des Siliconelastomers mit der INCI-Bezeichnung "Dimethicone Crosspolymer" in Isododecan), Dow Corning 9040 (Cyclomethicone, Dimethicone Crosspolymer), Dow Corning 9041 (Dimethicone, Dimethicone Crosspolymer) und Dow Corning 9045 (Cyclopentasiloxane, Dimethicone Crosspolymer).

Die erfindungsgemäßen Zusammensetzungen enthalten weiterhin, bezogen auf ihr Gesamtgewicht, 13 - 35 Gew.-% einer dispergierten Partikelphase. Bevorzugt sind 17 - 30 Gew.-%, außerordentlich bevorzugt 22 - 25 Gew.-%, jeweils bezogen auf das Gesamtgewicht der erfindungsgemäßen Zusammensetzung, an dispergierter Partikelphase enthalten.

Die Öltröpfchen der erfindungsgemäßen Öl-in-Wasser-Emulsion werden definitionsgemäß nicht als dispergierte Partikelphase verstanden. Unter dispergierter Partikelphase sind Partikel zu verstehen, die unter Normalbedingungen fest vorliegen und die weder in der wässrigen Phase noch in der Ölphase der erfindungsgemäßen Öl-in-Wasser-Emulsion löslich sind.

"Normalbedingungen" sind im Sinne der vorliegenden Anmeldung eine Temperatur von 20 °C und ein Druck von 1013,25 mbar. Schmelzpunktangaben beziehen sich ebenfalls auf einen Druck von 1013,25 mbar.

Die Bestandteile der Partikelphase weisen bevorzugt einen zahlenmittleren Partikeldurchmesser von 1 - 70 µm, bevorzugt 2 - 50 µm, besonders bevorzugt 5 - 30 µm und außerordentlich bevorzugt 10 - 25 µm, auf.

Ein wesentlicher Bestandteil dieser dispergierten Partikelphase ist ein Siliconelastomer, das in der wässrigen Phase oder in der Emulsion dispergiert vorliegt und nicht die Ölphase verdickt.

Ein derartiges Siliconelastomer ist wesentlich für das hervorragende Faltenkaschiervermögen der erfindungsgemäßen Zusammensetzung und verbessert weiterhin deren sensorische Eigenschaften. Das in der wässrigen Phase oder in der Emulsion dispergierte Siliconelastomer ist in einer Gesamtmenge von 13 - 35 Gew.-%, bezogen auf das Gesamtgewicht der erfindungsgemäßen Zusammensetzung, enthalten. Das bedeutet, dass die gesamte dispergierte Partikelphase aus Siliconelastomer(en) bestehen kann. Bevorzugt ist das in der wässrigen Phase oder in der Emulsion dispergierte Siliconelastomer in einer Gesamtmenge von 15 - 30 Gew.-%, außerordentlich bevorzugt 22 - 25 Gew.-%, jeweils bezogen auf das Gesamtgewicht der erfindungsgemäßen Zusammensetzung, enthalten.

Das in der wässrigen Phase oder in der Emulsion dispergierte Siliconelastomer kann in einer bevorzugten Ausführungsform der Erfindung in oberflächenmodifizierter Form vorliegen.

In einer besonders bevorzugten Ausführungsform der Erfindung liegt mindestens eine Teilmenge des enthaltenen Siliconelastomers b)i) in oberflächenmodifizierter Form vor. Diese Teilmenge beträgt bevorzugt 10 - 80 Gew.-%, besonders bevorzugt 30 - 60 Gew.-%, außerordentlich bevorzugt 40 - 50 Gew.-%, bezogen auf das Gesamtgewicht an in der wässrigen Phase oder in der Emulsion dispergiertem, nicht Ölphasen-verdickendem Siliconelastomer b)i).

In einer bevorzugten Ausführungsform ist das in der wässrigen Phase oder in der Emulsion dispergierte, nicht Ölphasen-verdickende Siliconelastomer b)i) an seiner Oberfläche mit Silica modifiziert.

Durch die Oberflächenmodifikation, insbesondere die Oberflächenmodifikation mit Silica, konnte das Faltenkaschiervermögen der erfindungsgemäßen Zusammensetzung in überraschendem Ausmaß weiter verbessert werden.

In einer weiteren besonders bevorzugten Ausführungsform der Erfindung liegen 10 - 80 Gew.-%, besonders bevorzugt 30 - 60 Gew.-%, außerordentlich bevorzugt 40 - 50 Gew.-%, bezogen auf das Gesamtgewicht an in der wässrigen Phase oder in der Emulsion dispergiertem, nicht Ölphasen-verdickendem Siliconelastomer b)i), in mit Silica oberflächenmodifizierter Form vor.

Eine weitere bevorzugte Ausführungsform der Erfindung ist dadurch gekennzeichnet, dass eine Teilmenge des Siliconelastomers b)i) der Emulsion als wässrige Vordispersion und eine weitere Teilmenge des Siliconelastomers b)i) als Pulver zugesetzt wird.

Mit dieser Aufteilung des Siliconelastomers b)i) gelang es überraschender Weise, das Faltenkaschiervermögen der erfindungsgemäßen Zusammensetzung noch weiter zu verbessern.

Die als Pulver zugesetzte Teilmenge beträgt bevorzugt 10 - 80 Gew.-%, besonders bevorzugt 30 - 60 Gew.-%, außerordentlich bevorzugt 40 - 50 Gew.-%, bezogen auf das Gesamtgewicht an in der wässrigen Phase oder in der Emulsion dispergiertem, nicht Ölphasen-verdickendem Siliconelastomer b)i).

Eine weitere bevorzugte Ausführungsform der Erfindung ist dadurch gekennzeichnet, dass nur die Teilmenge des Siliconelastomers b)i) oberflächenmodifiziert ist, die als Pulver zugesetzt wird. Eine weitere bevorzugte Ausführungsform der Erfindung ist dadurch gekennzeichnet, dass nur die Teilmenge des Siliconelastomers b)i) mit Silica oberflächenmodifiziert ist, die als Pulver zugesetzt wird.

Eine weitere bevorzugte Ausführungsform der Erfindung ist dadurch gekennzeichnet, dass 10 - 80 Gew.-%, besonders bevorzugt 30 - 60 Gew.-%, außerordentlich bevorzugt 40 - 50 Gew.-%, jeweils bezogen auf das Gesamtgewicht an in der wässrigen Phase oder in der Emulsion dispergiertem, nicht Ölphasen-verdickendem Siliconelastomer b)i), oberflächenmodifiziert ist und als Pulver zugesetzt wird.

Eine weitere bevorzugte Ausführungsform der Erfindung ist dadurch gekennzeichnet, dass 10 - 80 Gew.-%, besonders bevorzugt 30 - 60 Gew.-%, außerordentlich bevorzugt 40 - 50 Gew.-%, jeweils bezogen auf das Gesamtgewicht an in der wässrigen Phase oder in der Emulsion dispergiertem, nicht Ölphasen-verdickendem Siliconelastomer b)i), mit Silica oberflächenmodifiziert ist und als Pulver zugesetzt wird.

Eine weitere bevorzugte Ausführungsform der Erfindung ist dadurch gekennzeichnet, dass mindestens 70 Gew.-% der Siliconelastomerpartikel b)i) sphärisch sind. Bevorzugt beträgt der Anteil an sphärischen Siliconelastomerpartikeln b)i) mindestens 80 Gew.-%, besonders bevorzugt mindestens 90 Gew.-%, jeweils bezogen auf das Gesamtgewicht an in der wässrigen Phase oder in der Emulsion dispergiertem, nicht Ölphasen-verdickendem Siliconelastomer b)i). Es wurde festgestellt, dass das Faltenkaschiervermögen der erfindungsgemäßen Zusammensetzung umso besser ist, je höher der Anteil an sphärischen Siliconelastomerpartikeln b)i) ist.

Eine weitere bevorzugte Ausführungsform der Erfindung ist dadurch gekennzeichnet, dass die Partikelphase b) neben den Siliconelastomerpartikeln b)i) weitere Partikel enthält, die von Siliconelastomeren verschieden sind. Mit Hilfe derartiger Partikel können die sensorischen, mattierenden und Stabilitätseigenschaften der erfindungsgemäßen Zusammensetzung feinreguliert und weiter optimiert werden.

Eine weitere bevorzugte Ausführungsform der Erfindung ist dadurch gekennzeichnet, dass die von Siliconelastomeren verschiedenen Partikel ausgewählt sind aus folgenden organischen Materialien: Stärken und Stärkederivaten, die chemisch und/oder physikalisch modifiziert sein können, Cellulosepulvern, Polymerpulvern aus Polyolefinen, insbesondere Polyethylen-Pulvern und Polypropylen-Pulvern, Polycarbonaten, Polyurethanen, Polyamiden, Polyestern, Polystyrolen, Polyacrylaten, Polymethacrylaten, Polymethylmethacrylaten, (Meth)acrylat- oder (Meth)acrylat-Vinyliden-Copolymeren, Teflon oder Siliconen, sowie Mischungen hiervon.

Besonders bevorzugte organische Partikel sind ausgewählt aus Stärken und Stärkederivaten, die chemisch und/oder physikalisch modifiziert sein können, insbesondere modifizierten Stärkederivaten vom Typ der Stärkeoctenylsuccinatester, insbesondere der Aluminiumstärkeoctenylsuccinatester, beispielsweise erhältlich unter der Bezeichnung DRY FLO^{®} von National Starch, Cellulosepulvern, Polymerpulvern aus Polyolefinen, insbesondere Polyethylen-Pulvern und Polypropylen-Pulvern, Polycarbonaten, Polyurethanen, Polyamiden, Polyestern, Polystyrolen, Polyacrylaten, Polymethacrylaten, Polymethylmethacrylaten, (Meth)acrylat- oder (Meth)acrylat-Vinyliden-Copolymeren oder Teflon, wobei die Polymerpulver in einer besonders bevorzugten Ausführungsform der Erfindung vernetzt sein können, sowie Mischungen der genannten Substanzen.

Erfindungsgemäß besonders bevorzugte Polymerpulver auf Basis eines Polymethacrylat-Copolymers sind z. B. als Handelsprodukt Polytrap^{®} 6603 (Dow Corning) erhältlich. Andere erfindungsgemäß besonders bevorzugte Polymerpulver, z. B. auf Basis von Polyamiden, sind unter der Bezeichnung Orgasol^{®} 1002 (Polyamid-6) und Orgasol^{®} 2002 (Polyamid-12) von Elf Atochem erhältlich. Weitere erfindungsgemäß besonders bevorzugte Polymerpulver sind ausgewählt aus vernetzten Polymethacrylaten (insbesondere den Handelsprodukten Micropearl^{®} M von SEPPIC oder Plastic Powder A von NIKKOL), vernetzten Polymethylmethacrylaten (Micropearl^{®} M 305 und Micropearl^{®} M 310 von SEPPIC sowie Covabead von Cognis, insbesondere Covabead LH 85), Styrol-Divinylbenzol-Copolymeren (z. B. Plastic Powder FP von NIKKOL), Polyethylen- und Polypropylen-Pulvern (z. B. ACCUREL^{®} EP 400 von AKZO) oder auch Siliconpolymeren (z. B. Silicone Powder X2-1605 von Dow Corning).

Besonders bevorzugte partikelförmige Wirkstoffe sind ausgewählt aus vernetzten Polymethylmethacrylaten, bevorzugt mit der INCI "METHYL METHACRYLATE CROSSPOLYMER". Besonders bevorzugte vernetzte Polymethylmethacrylate weisen einen zahlenmittleren Partikeldurchmesser von 1-70 µm, bevorzugt 2-50 µm, besonders bevorzugt 5-30 µm und außerordentlich bevorzugt 10-25 µm auf. Außerordentlich bevorzugt sind Handelsprodukte Covabead LH 85 von Cognis und SUNPMMA-S von Sunjin.

Eine weitere bevorzugte Ausführungsform der Erfindung ist dadurch gekennzeichnet, dass mindestens 70 Gew.-% der von Siliconelastomeren verschiedenen Partikel sphärisch sind. Bevorzugt beträgt der Anteil an sphärischen Nicht-Siliconelastomerpartikeln mindestens 80 Gew.-%, besonders bevorzugt mindestens 90 Gew.-%, jeweils bezogen auf das Gesamtgewicht an von Siliconelastomeren verschiedenen Partikeln.

Weitere bevorzugte partikelförmige Wirkstoffe sind ausgewählt aus vernetzten Polymethylmethacrylaten mit zahlenmittleren Partikeldurchmessern von 0,5 - 50 µm, die halbkugelförmige Partikel mit Hohlräumen umfassen. Besonders bevorzugt sind mindestens 50 Gew.-% der Partikel halbkugelförmig mit Hohlräumen. Ein entsprechend besonders bevorzugtes Handelsprodukt ist Micropearl^{®} M 310 von SEPPIC.

Eine weitere bevorzugte Ausführungsform der Erfindung ist dadurch gekennzeichnet, dass 0,5 - 22 Gew.-%, bevorzugt 2-15 Gew.-%, besonders bevorzugt 3-10 Gew.-%, außerordentlich bevorzugt 5-8 Gew.-% an Partikeln, die von Siliconelastomeren verschieden sind, enthalten sind, wobei sich diese Mengenangaben auf das Gesamtgewicht der erfindungsgemäßen Zusammensetzung beziehen.

Die erfindungsgemäßen Zusammensetzungen enthalten 52,4 - 80,95 Gew.-%, bevorzugt 55 - 75 Gew.-%, besonders bevorzugt 60 - 70 Gew.-%, außerordentlich bevorzugt 63 - 68 Gew.-% an wässriger Phase, jeweils bezogen auf das Gesamtgewicht der erfindungsgemäßen Zusammensetzung.

Neben Wasser können in der wässrigen Phase bevorzugt wasserlösliche Polyole, die bevorzugt ausgewählt sind aus 1,2-Propylenglycol, Glycerin, 1,3-Butylenglycol, 1,6-Hexandiol, Hexylenglycol, 1,5-Pentandiol, Dipropylenglycol und unter Normalbedingungen flüssigen Polyethylenglycolen sowie Mischungen dieser Substanzen, enthalten sein. Weiterhin können diverse wasserlösliche kosmetische Wirkstoffe, wie Carnitin, Betain, Harnstoff, N,N'-Bis(2-hydroxyethyl)harnstoff, Ascorbinsäure und deren Salze sowie deren Phosphatester, wasserlösliche Alphahydroxycarbonsäuren und deren Salze, Ethanol, Proteinhydrolysate, Aminosäuren und deren Salze, und andere wasserlösliche Bestandteile in der wässrigen Phase enthalten sein.

Emulgatoren, insbesondere hydrophile Emulgatoren, werden erfindungsgemäß nicht zur wässrigen Phase gerechnet.

Die erfindungsgemäßen Zusammensetzungen enthalten mindestens einen Emulgator, wobei insgesamt 0,05 bis 0,6 Gew.-% Emulgator(en), bezogen auf das Gesamtgewicht der erfindungsgemäßen Zusammensetzung, enthalten sind. Bevorzugt beträgt der Gesamtgehalt an Emulgator(en), jeweils bezogen auf das Gesamtgewicht der erfindungsgemäßen Zusammensetzung, 0,1 - 0,5 Gew.-%, besonders bevorzugt 0,2 -0,4 Gew.-%.

Bevorzugt ist mindestens ein Öl-in-Wasser-Emulgator enthalten. Besonders bevorzugt beträgt der Gesamtgehalt an Öl-in-Wasser-Emulgator(en), jeweils bezogen auf das Gesamtgewicht der erfindungsgemäßen Zusammensetzung, 0,05 bis 0,6 Gew.-%, besonders bevorzugt 0,1 - 0,5 Gew.-%, außerordentlich bevorzugt 0,2 - 0,4 Gew.-%.

Bevorzugte Öl-in-Wasser-Emulgatoren sind ausgewählt sind aus ethoxylierten C₈-C₂₄-Alkanolen mit durchschnittlich 5-100 Mol Ethylenoxid pro Mol, ethoxylierten C₈-C₂₄-Carbonsäuren mit durchschnittlich 5-100 Mol Ethylenoxid pro Mol, Silicon-Copolyolen mit Ethylenoxid-Einheiten oder mit Ethylenoxid- und Propylenoxid-Einheiten, Alkylmono- und -oligoglycosiden mit 8 bis 22 Kohlenstoffatomen im Alkylrest und deren ethoxylierten Analoga, ethoxylierten Sterinen, Partialestern von Polyglycerinen mit 2 bis 10 Glycerineinheiten und mit 1 bis 4 gesättigten oder ungesättigten, linearen oder verzweigten, gegebenenfalls hydroxylierten C₈-C₃₀-Fettsäureresten verestert, sofern sie einen HLB-Wert von mehr als 7 aufweisen, sowie Mischungen der vorgenannten Substanzen.

Die ethoxylierten C₈-C₂₄-Alkanole haben die Formel R¹O(CH₂CH₂O)ₙH, wobei R¹ steht für einen linearen oder verzweigten Alkyl- und/oder Alkenylrest mit 8-24 Kohlenstoffatomen und n, die mittlere Anzahl der Ethylenoxid-Einheiten pro Molekül, für Zahlen von 5 - 100, vorzugsweise 10-30 Mol Ethylenoxid an 1 Mol Caprylalkohol, 2-Ethylhexylalkohol, Caprinalkohol, Laurylalkohol, Isotridecylalkohol, Myristylalkohol, Cetylalkohol, Palmitoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol sowie deren technische Mischungen. Auch Addukte von 5-100 Mol, vorzugsweise 10-30 Mol, Ethylenoxid an technische Fettalkohole mit 12-18 Kohlenstoffatomen, wie beispielsweise Kokos-, Palm-, Palmkern- oder Talgfettalkohol, sind geeignet. Besonders bevorzugte ÖI-in-Wasser-Emulgatoren sind ausgewählt aus der Gruppe, bestehend aus Ceteth-12, Ceteth-20, Ceteth-30, Steareth-12, Steareth-20, Steareth-30, Laureth-12 und Beheneth-20, sowie Mischungen hiervon.

Die ethoxylierten C₈-C₂₄-Carbonsäuren haben die Formel R¹(OCH₂CH₂)ₙOH, wobei R¹ steht für einen linearen oder verzweigten gesättigten oder ungesättigten Acylrest mit 8 -24 Kohlenstoffatomen und n, die mittlere Anzahl der Ethylenoxid-Einheiten pro Molekül, für Zahlen von 5- 100, vorzugsweise 10-30 Mol Ethylenoxid an 1 Mol Caprylsäure, 2-Ethylhexansäure, Caprinsäure, Laurinsäure, Isotridecansäure, Myristinsäure, Cetylsäure, Palmitoleinsäure, Stearinsäure, Isostearinsäure, Ölsäure, Elaidinsäure, Petroselinsäure, Arachinsäure, Gadoleinsäure, Behensäure, Erucasäure und Brassidinsäure sowie deren technische Mischungen. Auch Addukte von 5-100 Mol, vorzugsweise 10-30 Mol, Ethylenoxid an technische Fettsäuren mit 12 - 18 Kohlenstoffatomen, wie Kokos-, Palm-, Palmkern- oder Talgfettsäure, sind geeignet. Besonders bevorzugt sind PEG-50-monostearat, PEG-100-monostearat, PEG-50-monooleat, PEG-100-monooleat, PEG-50-monolaurat und PEG-100-monolaurat.

Besonders bevorzugt eingesetzt werden die C₁₂-C₁₈-Alkanole oder die C₁₂-C₁₈-Carbonsäuren mit jeweils 10-30 Einheiten Ethylenoxid pro Molekül sowie Mischungen dieser Substanzen.

Weiterhin werden vorzugsweise C₈-C₂₂-Alkylmono- und -oligoglycoside eingesetzt. C₈-C₂₂-Alkylmono- und -oligoglycoside stellen bekannte, handelsübliche ÖI-in-Wasser-Emulgatoren dar. Ihre Herstellung erfolgt insbesondere durch Umsetzung von Glucose oder Oligosacchariden mit primären Alkoholen mit 8-22 Kohlenstoffatomen. Bezüglich des Glycosidrestes gilt, dass sowohl Monoglycoside, bei denen ein cyclischer Zuckerrest glycosidisch an den Fettalkohol gebunden ist, als auch oligomere Glycoside mit einem Oligomerisationsgrad bis etwa 8, vorzugsweise 1 - 2, geeignet sind. Der Oligomerisierungsgrad ist dabei ein statistischer Mittelwert, dem eine für solche technischen Produkte übliche Homologenverteilung zugrunde liegt. Produkte, die unter dem Warenzeichen Plantacare^{®} erhältlich sind, enthalten eine glucosidisch gebundene C₈-C₁₆-Alkylgruppe an einem Oligoglucosidrest, dessen mittlerer Oligomerisationsgrad bei 1-2 liegt. Besonders bevorzugte C₈-C₂₂-Alkylmono- und -oligoglycoside sind ausgewählt aus Octylglucosid, Decylglucosid, Laurylglucosid, Palmitylglucosid, Isostearylglucosid, Stearylglucosid, Arachidylglucosid und Behenylglucosid sowie Mischungen hiervon. Auch die vom Glucamin abgeleiteten Acylglucamide sind als nicht-ionische Öl-in-Wasser-Emulgatoren geeignet.

Auch ethoxylierte Sterine, insbesondere ethoxylierte Sojasterine, stellen erfindungsgemäß geeignete Öl-in-Wasser-Emulgatoren dar. Der Ethoxylierungsgrad muss größer als 5, bevorzugt mindestens 10 sein, um einen HLB-Wert größer 7 aufzuweisen. Geeignete Handelsprodukte sind z. B. PEG-10 Soy Sterol, PEG-16 Soy Sterol und PEG-25 Soy Sterol.

Weiterhin werden vorzugsweise Partialester von Polyglycerinen mit 2 bis 10 Glycerineinheiten und mit 1 bis 4 gesättigten oder ungesättigten, linearen oder verzweigten, gegebenenfalls hydroxylierten C₈-C₃₀-Fettsäureresten verestert, eingesetzt, sofern sie einen HLB-Wert von mehr als 7 aufweisen. Besonders bevorzugt sind Diglycerinmonocaprylat, Diglycerinmonocaprat, Diglycerinmonolaurat, Triglycerinmonocaprylat, Triglycerinmonocaprat, Triglycerinmonolaurat, Tetraglycerinmonocaprylat, Tetraglycerinmonocaprat, Tetraglycerinmonolaurat, Pentaglycerinmonocaprylat, Pentaglycerinmonocaprat, Pentaglycerinmonolaurat, Hexaglycerinmonocaprylat, Hexaglycerinmonocaprat, Hexaglycerinmonolaurat, Hexaglycerinmonomyristat, Hexaglycerinmonostearat, Decaglycerinmonocaprylat, Decaglycerinmonocaprat, Decaglycerinmonolaurat, Decaglycerinmonomyristat, Decaglycerinmonoisostearat, Decaglycerinmonostearat, Decaglycerinmonooleat, Decaglycerinmonohydroxystearat, Decaglycerindicaprylat, Decaglycerindicaprat, Decaglycerindilaurat, Decaglycerindimyristat, Decaglycerindiisostearat, Decaglycerindistearat, Decaglycerindioleat, Decaglycerindihydroxystearat, Decaglycerintricaprylat, Decaglycerintricaprat, Decaglycerintrilaurat, Decaglycerintrimyristat, Decaglycerintriisostearat, Decaglycerintristearat, Decaglycerintrioleat und Decaglycerintrihydroxystearat.

Daneben können die erfindungsgemäßen Zusammensetzungen auch - in Bezug auf die Gesamtmenge an Emulgator - untergeordnete Mengen an mindestens einem Wasser-in-Öl-Emulgator enthalten, beispielsweise Sorbitanoleat. Bevorzugt ist hierbei ein Gesamtgehalt an Wasser-in-ÖI-Emulgator(en) von 0 - 0,3 Gew.-%, besonders bevorzugt 0,01 - 0,1 Gew.-%, außerordentlich bevorzugt 0,02 - 0,06 Gew.-%, jeweils bezogen auf das Gesamtgewicht der erfindungsgemäßen Zusammensetzung, enthalten.

Die erfindungsgemäßen Zusammensetzungen sind frei von Wachsen, Alkanolen und Polyolestern, die jeweils einen Schmelzpunkt von 30°C und darüber aufweisen. Es wurde festgestellt, dass derartige Substanzen das Faltenkaschiervermögen der erfindungsgemäßen Zusammensetzungen negativ beeinflussen können.

Entsprechende Substanzen mit einem Schmelzpunkt von 30°C und darüber sind beispielsweise natürliche pflanzliche Wachse, z. B. Candelillawachs, Carnaubawachs, Japanwachs, Zuckerrohrwachs, Ouricourywachs, Korkwachs, Sonnenblumenwachs, Fruchtwachse wie Orangenwachse, Zitronenwachse, Grapefruitwachs, und tierische Wachse, z. B. Bienenwachs und Schellackwachs, hydrierte oder gehärtete Wachse, chemisch modifizierte Wachse, insbesondere die Hartwachse, wie z. B. Montanesterwachse, hydrierte Jojobawachse und Sasolwachse, synthetische Wachse, beispielsweise Polyalkylenwachse und Polyethylenglycolwachse, C₂₀-C₄₀-Dialkylester von Dimersäuren, C₃₀₋₅₀-Alkylbienenwachs sowie Alkyl- und Alkylarylester von Dimerfettsäuren, Ester aus einem gesättigten, einwertigen C₁₂-C₆₀-Alkanol und einer gesättigten C₈-C₃₆-Monocarbonsäure, C₁₆-₃₆-Alkylstearate, C₁₈-₃₈-Alkylhydroxystearoylstearate, C₂₀-₄₀-Alkylerucate, Cetearylbehenat, die Triglyceride gesättigter und gegebenenfalls hydroxylierter C₁₂₋₃₀-Fettsäuren, wie gehärtete Triglyceridfette (hydriertes Palmöl, hydriertes Kokosöl, hydriertes Rizinusöl), Glyceryltribehenat (Tribehenin) oder Glyceryltri-12-hydroxystearat, weiterhin synthetische Vollester aus Fettsäuren und Glycolen oder Polyolen mit 2- 6 Kohlenstoffatomen, solange sie einen Schmelzpunkt oberhalb von 50°C aufweisen, beispielsweise C₁₈-C₃₆ Acid Triglyceride (Syncrowax^{®} HGL-C), hydriertes Rizinusöl, die gesättigten linearen C₁₄-C₃₆-Carbonsäuren, insbesondere Myristinsäure, Palmitinsäure, Stearinsäure und Behensäure, die gesättigten linearen Alkanole mit mindestens 10 Koh-Ienstoffatomen, wie Caprinalkohol, Laurylalkohol, Isotridecylalkohol, Myristylalkohol, Cetylalkohol, Palmitoleylalkohol, Stearylalkohol, Isostearylalkohol, Oleylalkohol, Elaidylalkohol, Petroselinylalkohol, Arachylalkohol, Gadoleylalkohol, Behenylalkohol, Erucylalkohol und Brassidylalkohol, weiterhin Polyolester, wie Glycerinmonolaurat, Glycerinmonostearat, Glycerindistearat usw..

Ein weiterer Gegenstand der vorliegenden Anmeldung ist die nicht-therapeutische Verwendung einer kosmetischen Zusammensetzung gemäß Anspruch 1, 2, 3, 4, 5, 6, 7, 8 oder 9 zur optischen Kaschierung von Falten und Unregelmäßigkeiten der Haut und/oder zur Mattierung der Haut und/oder zum Egalisieren der Hautfarbe.

Bezüglich bevorzugter Ausführungsformen der erfindungsgemäßen Verwendung gilt mutatis mutandis das zu den erfindungsgemäßen Zusammensetzungen Gesagte.

Ein weiterer Gegenstand der vorliegenden Anmeldung ist ein nicht-therapeutisches Verfahren zur optischen Kaschierung von Falten und Unregelmäßigkeiten der Haut und/oder zur Mattierung der Haut und/oder zum Egalisieren der Hautfarbe, bei dem eine kosmetische Zusammensetzung gemäß Anspruch 1, 2, 3, 4, 5, 6, 7, 8 oder 9 auf die Haut aufgetragen wird.

Bezüglich bevorzugter Ausführungsformen des erfindungsgemäßen Verfahrens gilt mutatis mutandis das zu den erfindungsgemäßen Zusammensetzungen Gesagte.

Ein weiterer Gegenstand der vorliegenden Anmeldung ist ein Verfahren zur Herstellung einer erfindungsgemäßen oder erfindungsgemäß bevorzugten Öl-in-Wasser-Emulsion, das die folgenden Verfahrensschritte umfasst:
i. Bereitstellen einer Ölphase (a) durch Vermischen aller Bestandteile der Ölphase mit Ausnahme des Parfüms,
ii. Bereitstellen einer wässrigen Dispersion (wdP) von Siliconelastomerpartikeln,
iii. Vermischen der Ölphase (a) mit der wässrigen Dispersion (wdP) von Siliconelastomerpartikeln und Homogenisierung dieser Mischung (a) und (wdP) zu einer Öl-in-Wasser-Voremulsion (ve),
iv. Bereitstellen einer wässrigen Phase (c) durch Vermischen aller Bestandteile der wässrigen Phase mit Ausnahme von Verdickungs- und Konservierungsmitteln,
v. Vermischen der wässrigen Phase (c) mit der Voremulsion (ve) und Homogenisierung dieser Mischung (c) und (ve) zur Öl-in-Wasser-Emulsion (owe),
vi. Zumischung von Parfüm, Verdickungs- und Konservierungsmitteln zur Öl-in-Wasser-Emulsion (owe) und
vii. Zumischung von pulverförmigen Siliconelastomerpartikeln zur Öl-in-Wasser-Emulsion (owe).

In einer bevorzugten Ausführungsform ist das erfindungsgemäße Herstellungsverfahren dadurch gekennzeichnet, dass alle Verfahrensschritte bei einer Temperatur von 15 - 30 °C, bevorzugt 18 - 25 °C, ablaufen. Dies bedeutet, dass das erfindungsgemäße Herstellungsverfahren bevorzugt bei Raumtemperatur durchgeführt wird, ohne dass in den einzelnen Verfahrensschritten Wärme zugeführt wird.

In einer weiteren bevorzugten Ausführungsform ist das erfindungsgemäße Herstellungsverfahren dadurch gekennzeichnet, dass die in Verfahrensschritt vii. zugemischten Siliconelastomerpartikel mit Silica oberflächenmodifiziert sind.

In einer weiteren bevorzugten Ausführungsform ist das erfindungsgemäße Herstellungsverfahren dadurch gekennzeichnet, dass im Verfahrensschritt vii. zusätzlich zu den Siliconelastomerpartikeln Partikel zugemischt werden, die von Siliconelastomeren verschieden sind.

Bezüglich weiterer bevorzugter Ausführungsformen des erfindungsgemäßen Herstellungsverfahrens gilt mutatis mutandis das zu den erfindungsgemäßen Zusammensetzungen Gesagte.

Die folgenden Beispiele sollen die Erfindung verdeutlichen, ohne sie hierauf zu beschränken. Die Mengenangaben sind in Gew.-%.

### Beispiel 1: Öl-in-Wasser-Emulsion mit sofort eintretendem Faltenkaschiereffekt

| | | |
|---|---|---|
| Dimethicone/Vinyl Dimethicone Crosspolymer(zahlenmittlerer Partikeldurchmesser: 3 µm) | 15 (active matter) | Partikelphase Siliconelastomer, in wässriger Vordispersion eingesetzt |
| Dimethicone/Vinyl Dimethicone Crosspolymer, Silica (zahlenmittlerer Partikeldurchmesser: 1-10 µm) | 13 | Partikelphase (mit Silica oberflächenmodifiziertes Siliconelastomer) |
| Methyl Methacrylate Crosspolymer (zahlenmittlerer Partikeldurchmesser: 5-10 µm) | 4 | Partikelphase |
| Silica Dimethyl Silylate | 0,2 | Partikelphase |
| 1,6-Hexandiol | 6 | wässrige Phase |
| Glycerin | 6 | wässrige Phase |
| 1,3-Butylenglycol | 3 | wässrige Phase |
| N,N'-Bis(2-hydroxyethyl)harnstoff | 2,0 | wässrige Phase |
| C12-15 Alkyl Benzoate | 6 | Ölphase |
| Dimethicone (5 cSt) | 1,0 | Ölphase |
| Cyclomethicone | 1,0 | Ölphase |
| Polyisobutene | 0,8 | Ölphase |
| 2-Ethylhexylpalmitat | 2 | Ölphase |
| Dimethicone Crosspolymer | 0,6 | Ölphase |
| C12-14 Pareth-12 | 0,2 | O/W-Emulgator |
| Isoceteth-10 | 0,3 | O/W-Emulgator |
| Sodium Acrylate/Sodium Acryloyldimethyl Taurate Copolymer | 1,0 | wässrige Phase |
| Carbomer | 0,1 | wässrige Phase |
| Sorbitanoleat | 0,1 | W/O-Emulgator |
| Caprylyl/Capryl Glucoside | 0,1 | O/W-Emulgator |
| Parfum | 0,3 | Ölphase |
| Phenoxyethanol | 0,5 | wässrige Phase |
| Methylparaben | 0,1 | wässrige Phase |
| Ethylparaben | 0,1 | wässrige Phase |
| Aqua | ad 100 | wässrige Phase |

Die Bestandteile der Ölphase (mit Ausnahme des Parfums) und der wässrigen Phase (mit Ausnahme der Verdickerbestandteile Sodium Acrylate/Sodium Acryloyldimethyl Taurate Copolymer, Sorbitanoleat, Caprylyl/Capryl Glucoside und Carbomer und der Konservierungsmittel) werden bei Raumtemperatur getrennt voneinander gemischt. Anschließend wird zu der vorgemischten Ölphase die Hälfte des Siliconelastomers b)i) (= Dimethicone/ Vinyl Dimethicone Crosspolymer) in Form einer wässrigen Vordispersion zugegeben und homogenisiert. Direkt im Anschluss wird die wässrige Phase hinzugegeben und die Emulsion wird homogenisiert. Unter Rühren werden nun die Konservierungsmittel, das Parfum und die Verdickerbestandteile (Sodium Acrylate/ Sodium Acryloyldimethyl Taurate Copolymer, Sorbitanoleat, Caprylyl/Capryl Glucoside und Carbomer) untergemischt. Zum Schluss werden die zweite Hälfte des Siliconelastomers b)i) (= Dimethicone/Vinyl Dimethicone Crosspolymer und Silica), das mit Silica oberflächenmodifiziert ist, und die Methyl Methacrylate Crosspolymer-Partikel zugegeben und vermischt.

Bei Applikation auf die faltige Haut im Augenbereich eines Probandinnen-Panels von 30 Frauen im Alter von 48 bis 60 Jahren zeigte sich mittels berührungsloser FOITS-Untersuchungen (Fast Optical In Vivo Topometry of Human Skin) nach 1 Minute, 1 Stunde bzw. 2 Stunden nach Applikation im Vergleich zum jeweils unbehandelten anderen Augenbereich eine deutliche optische Reduktion der Faltentiefe:

| | Value of roughness Rz (difference to initial value) | Depth of roughness Ra (difference to initial value) | Value of roughness Rz (difference corrected by untreated area) | Depth of roughness Ra (difference corrected by untreated area) |
|---|---|---|---|---|
| 1 minute after treatment | -19,5 % | -20,9 % | --- | --- |
| 1 hour after treatment | -15,7% | -16,7% | -14,0 % | -14,8% |
| 2 hours after treatment | -15,6 % | -16,3% | -13,7% | -14,3% |

### Beispiel 2: Öl-in-Wasser-Emulsion mit sofort eintretendem Faltenkaschiereffekt

| | | |
|---|---|---|
| Dimethicone/Vinyl Dimethicone Crosspolymer(zahlenmittlerer Partikeldurchmesser: 3 µm) | 10 (active matter) | Partikelphase Siliconelastomer, in wässriger Vordispersion eingesetzt |
| Dimethicone/Vinyl Dimethicone Crosspolymer, Silica (zahlenmittlerer Partikeldurchmesser: 1-10) µm) | 8 | Partikelphase (mit Silica oberflächenmodifiziertes Siliconelastomer) |
| 1,6-Hexandiol | 6 | wässrige Phase |
| Glycerin | 6 | wässrige Phase |
| 1,3-Butylenglycol | 3 | wässrige Phase |
| C12-15 Alkyl Benzoate | 7,5 | Ölphase |
| Dimethicone (5 cSt) | 1,0 | Ölphase |
| Cyclomethicone | 1,0 | Ölphase |
| Squalane | 0,8 | Ölphase |
| Dimethicone Crosspolymer | 0,6 | Ölphase |
| C12-14 Pareth-12 | 0,2 | O/W-Emulgator |
| Isoceteth-10 | 0,3 | O/W-Emulgator |
| Hydroxyethylacrylate/Sodium Acryloyldimethyl Taurate Copolymer | 1,2 | wässrige Phase |
| Polysorbate 60 | 0,1 | O/W-Emulgator |
| Parfum | 0,3 | Ölphase |
| Phenoxyethanol | 0,5 | wässrige Phase |
| Methylparaben | 0,1 | wässrige Phase |
| Ethylparaben | 0,1 | wässrige Phase |
| Aqua | ad 100 | wässrige Phase |

Diese Öl-in-Wasser-Emulsion wurde analog zu Beispiel 1 hergestellt.

## Patentansprüche

1. Kosmetische Zusammensetzung in Form einer Öl-in-Wasser-Emulsion, enthaltend, jeweils bezogen auf das Gesamtgewicht der Zusammensetzung:
a) 6-12 Gew.-% Ölphase, darin
a)i) 0,1 - 0,7 Gew.-% Siliconelastomer, das die Ölphase verdickt,
a)ii) 0 bis maximal 4 Gew.-% Siliconöl,
b) 13-35 Gew.-% dispergierte Partikelphase, darin
b)i) 13-35 Gew.-% Siliconelastomer, das in der wässrigen Phase oder in der Emulsion dispergiert vorliegt und nicht die Ölphase verdickt,
c) 52,4 - 80,95 Gew.-% wässrige Phase,
d) wobei die Zusammensetzung insgesamt 0,05 bis 0,6 Gew.-% Emulgator(en) enthält, und
e) wobei die Zusammensetzung frei ist von Wachsen, Alkanolen und Polyolestern, die jeweils einen Schmelzpunkt von 30°C und darüber aufweisen.

2. Kosmetische Zusammensetzung gemäß Anspruch 1, **dadurch gekennzeichnet, dass** mindestens eine Teilmenge des Siliconelastomers b)i) in oberflächenmodifizierter Form eingesetzt wird.

3. Kosmetische Zusammensetzung gemäß Anspruch 2, **gekennzeichnet durch** Silica als Oberflächenmodifikationsmittel für das Siliconelastomer b)i).

4. Kosmetische Zusammensetzung gemäß Anspruch 1, 2 oder 3, **dadurch gekennzeichnet, dass** eine Teilmenge des Siliconelastomers b)i) als wässrige Vordispersion und eine weitere Teilmenge des Siliconelastomers b)i) als Pulver zugesetzt wird.

5. Kosmetische Zusammensetzung gemäß Anspruch 2, 3 oder 4, **dadurch gekennzeichnet, dass** nur die Teilmenge des Siliconelastomers b)i) oberflächenmodifiziert ist, die als Pulver zugesetzt wird.

6. Kosmetische Zusammensetzung gemäß Anspruch 1, 2, 3, 4 oder 5, **dadurch gekennzeichnet, dass** mindestens 70 Gew.-% der Siliconelastomerpartikel b)i), bezogen auf das Gesamtgewicht an Siliconelastomer b)i), sphärisch sind.

7. Kosmetische Zusammensetzung gemäß Anspruch 1, 2, 3, 4, 5 oder 6, **dadurch gekennzeichnet, dass** die Partikelphase b) Partikel enthält, die von Siliconelastomeren verschieden sind.

8. Kosmetische Zusammensetzung gemäß Anspruch 7, **dadurch gekennzeichnet, dass** die Partikelphase b) Partikel enthält, die ausgewählt sind aus folgenden organischen Materialien: Stärken und Stärkederivaten, die chemisch und/oder physikalisch modifiziert sein können, Cellulosepulvern, Polymerpulvern aus Polyolefinen, insbesondere Polyethylen-Pulvern und Polypropylen-Pulvern, Polycarbonaten, Polyurethanen, Polyamiden, Polyestern, Polystyrolen, Polyacrylaten, Polymethacrylaten, Polymethylmethacrylaten, (Meth)acrylat- oder (Meth)acrylat-Vinyliden-Copolymeren, Teflon oder Siliconen, sowie Mischungen hiervon.

9. Kosmetische Zusammensetzung gemäß Anspruch 1, 2, 3, 4, 5, 6, 7 oder 8, **dadurch gekennzeichnet, dass** die Ölphase ein Esteröl enthält.

10. Nicht-therapeutische Verwendung einer kosmetischen Zusammensetzung gemäß Anspruch 1, 2, 3, 4, 5, 6, 7, 8 oder 9 zur optischen Kaschierung von Falten und Unregelmäßigkeiten der Haut und/oder zur Mattierung der Haut und/oder zum Egalisieren der Hautfarbe.

11. Nicht-therapeutisches Verfahren zur optischen Kaschierung von Falten und Unregelmäßigkeiten der Haut und/oder zur Mattierung der Haut und/oder zum Egalisieren der Hautfarbe, **dadurch gekennzeichnet, dass** eine kosmetische Zusammensetzung gemäß Anspruch 1, 2, 3, 4, 5, 6, 7, 8 oder 9 auf die Haut aufgetragen wird.

12. Verfahren zur Herstellung einer Öl-in-Wasser-Emulsion gemäß einem der Ansprüche 1-9, das die folgenden Verfahrensschritte umfasst:
i. Bereitstellen einer Ölphase (a) durch Vermischen aller Bestandteile der Ölphase mit Ausnahme des Parfüms,
ii. Bereitstellen einer wässrigen Dispersion (wdP) von Siliconelastomerpartikeln,
iii. Vermischen der Ölphase (a) mit der wässrigen Dispersion (wdP) von Siliconelastomerpartikeln und Homogenisierung dieser Mischung (a) und (wdP) zu einer Öl-in-Wasser-Voremulsion (ve),
iv. Bereitstellen einer wässrigen Phase (c) durch Vermischen aller Bestandteile der wässrigen Phase mit Ausnahme von Verdickungs- und Konservierungsmitteln,
v. Vermischen der wässrigen Phase (c) mit der Voremulsion (ve) und Homogenisierung dieser Mischung (c) und (ve) zur Öl-in-Wasser-Emulsion (owe),
vi. Zumischung von Parfüm, Verdickungs- und Konservierungsmitteln zur Öl-in-Wasser-Emulsion (owe) und
vii. Zumischung von pulverförmigen Siliconelastomerpartikeln zur Öl-in-Wasser-Emulsion (owe).

13. Verfahren nach Anspruch 12, **dadurch gekennzeichnet, dass** alle Verfahrensschritte bei einer Temperatur von 15-30°C, bevorzugt 18-25°C, ablaufen.

14. Verfahren nach Anspruch 12 oder 13, **dadurch gekennzeichnet, dass** die in Verfahrensschritt vii. zugemischten Siliconelastomerpartikel mit Silica oberflächenmodifiziert sind.

15. Verfahren nach Anspruch 12, 13 oder 14, **dadurch gekennzeichnet, dass** im Verfahrensschritt vii. Partikel zugemischt werden, die von Siliconelastomeren verschieden sind.
